(19) Europäisches Patentamt
European Patent Office
Office européen des brevets

(11) Publication number : **0 409 903 B1**

(12) **EUROPEAN PATENT SPECIFICATION**

(45) Date of publication of patent specification : **29.04.92 Bulletin 92/18**

(51) Int. Cl.$^5$ : **C11C 1/10,** C11C 1/02, C07C 51/44, C07C 57/03, A61K 31/20, A23L 1/30

(21) Application number : **89906102.2**

(22) Date of filing : **24.05.89**

(86) International application number : **PCT/EP89/00577**

(87) International publication number : **WO 89/11521 30.11.89 Gazette 89/28**

# (54) PROCESS FOR PREPARING POLYUNSATURATED FATTY ACIDS.

(30) Priority : **27.05.88 GB 8812655**

(43) Date of publication of application : **30.01.91 Bulletin 91/05**

(45) Publication of the grant of the patent : **29.04.92 Bulletin 92/18**

(84) Designated Contracting States : **AT BE CH DE FR GB IT LI LU NL SE**

(56) References cited :
**EP-A- 0 292 846**
**WO-A-88/08444**
**GB-A- 2 090 529**
**GB-A- 2 152 043**
**US-A- 3 082 228**
**US-A- 4 554 107**

(56) References cited :
**Derwent File Supplier WPI(L) 1988, AN - 88-145139(21) Derwent Publications Ltd (London; GB) & JP, A, 63088159 (NIPPON OILS & FATS K.K.) 19 April 1988, see abstract.**
**Derwent File Supplier WPI(L) 1984 AN - 84-198209 (32) Derwent Publications Ltd (London, GB) & JP, A, 59113099 (OISHI K) 29 June 1984, see abstract.**

(73) Proprietor : **STAROIL LIMITED**
**No. 4 Columbus Centre Wickhams Cay Road Town**
**British Virgin Islands (GB)**

(72) Inventor : **MOSCA TENNA, Giovanni**
**Elektrastrasse 38**
**W-8000 München 81 (DE)**
Inventor : **ZIGERLIG, Max**
**Casa Torre, 1**
**Via Valle Brere CH-6598 Tenero (CH)**

(74) Representative : **Minoja, Fabrizio**
**Studio Consulenza Brevettuale Via Rossini, 8**
**I-20122 Milano (IT)**



Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a process for preparing a high-concentration mixture of eicosapentaenoic acid and docosahexa/enoic acid, and of their esters, by starting from oils of various animal and/or vegetable origins, as well as to the so obtained mixtures.

The process of the present invention is furthermore suitable for deodourising and deacidifying the same oils, in view of a possible dietetical or alimentary use thereof.

It is known by now that the polyunsaturated fatty acids play an important role in human being physiology, because they perform, in particular, two roles; a structural role, as constituents of the phospholipids of the cellular membranes, and a functional role, as precursors of prostaglandins.

The fatty acids belonging to the family of α-linolenic acid perform in fact a basic task for the development and function of brain, retina and gonads, as well as for the formation of $PGI_3$ and $TXA_3$, extremely important factors for platelet agglutination preventive effect.

Among these, in particular, important are the long-chain members of ω-3 family, i.e., eicosapentaenoic acid (20:5ω-3), or EPA, and docosahexaenoic acid (22:6ω3), or DHA, which derive from the desaturation and chain extension of α-linolenic acid, thanks to the intervention of the relevant enzymes (Δ-desaturases).

EPA, as a precursor of both $PGI_3$ and $TxA_3$, performs a platelet agglutination prevention action and an antithrombotic effect which can be reconducted to an inhibition of cyclcoxygenase (an aspirin-like effect) and/or to the competition with arachidonic acid for this enzyme, with a consequent decreased synthesis of $PGE_2$ and $TxA_2$, well-known platelet agglutinants.

DHA is the most important component of brain lipids in man and is present at high concentrations in the phospholipids of the synaptic membranes, a fact, this, which makes researchers suppose DHA to play a role in the transmission of nervous impulse.

Furthermore, inasmuch as it is a structural element of platelet cell, DHA indirectly plays, by increasing platelet fluidity, an important role in antithrombotic action.

Recent studies on man evidenced a decrease of Δ -6-desaturase enzyme with increasing age (after 35 years of age); as a consequence, an endogeous deficiency could occur of above said acids, which therefore should be administered by means of the diet, or by means of suitable compositions. However, to date several practical difficulties have prevented a wide use of said acids to be made in therapy or as alimentary integrators, a use which, on the other hand, would be highly desirable in view of the above reported biochemical and pharmacological background. Such difficulties are mainly related to the extraction of said acids from fish oils, their purification and concentration up to suitable values for a pharmaceutical use, and their adourisation.

Although many methods have been proposed and published in the past, the above cited objectives have not been reached to a satisfactory extent yet, as, among others, the still now limited use of EPA and/or DHA demonstrates, notwithstanding their considerable potentialities as drugs or alimentary integrators. The methods known to date, based on different techniques, such as degreasing, counter-current extraction, urea addition, liquid chromatography, distillation, lead to rather low yields and to easily perishable products if exposed to light or to atmosphere. Furthermore, most known methods aim at purifying eicosapentaenoic acid only, to the detriment of other useful unsaturated fatty acids, such as DHA.

For example, U.S. patent 4,377,526 discloses a process for purifying EPA, or its esters, which comprises a treatment with urea, followed by fractional distillation. By such a method, percentages of EPA higher than 70% are obtained, whilst DHA remains present as a residue (3-5%) only.

Furthermore, as far as the present Applicant knows to date, all patented processes relating to the production of the same, or of similar, products, use a more or less complex combination of chemical or physical operations, such as, e.g., the use of urea for the preferential precipitation of less unsaturated acids (WO 87/03899, JP 57-187397), or extractions with supercritical fluids (JP 60-214757, JP 60-115698).

In order to reach high titers of DHA acids, or of esters thereof, in other patents also chromatography is used, with various chromatographic beds which range fron silica gel up to low-polarity copolymers (JP 61-291540, JP 61-037752, JP 58-109444, GB 2090529).

On the other hand, in those patents in which the molecular distillation is used (as, e.g., JP-113099 GB-A-2090529), this is not the characterizing step of the process, but is simply used as a means for a rough purification during the processing.

The process of the present invention is exclusively based, apart from the common hydrolysis of triglycerides in order to obtain the acids, on the technique of molecular distillation. The molecular distillation is used by suitably changing the operating conditions, in order to obtain the whole range of products of the present invention, without any other chemical or physical treatments.

The object of the present invention is hence a method for extracting DHA and EPA ethyl esters and free fatty acids from raw oils of various kinds with high yields, under conditions easily applicable in the industrial field, and leading to a stable and odourless product, which can be used in human therapy both as a pharmaceutical and as a dietetic and alimentary product.

Furthermore, such a process only requires a very small number of chemical treatments, in that it is substantially based on the articulated use of a technology, i.e., molecular distillation, which, owing to its operating conditions, secures the highest protection of the products of the invention. In fact, these products are known to be very subject to undergo phenomena of chemical and thermal degradation. Finally, from the standpoint of the industrial economic feasibility, molecular distillation is per se suitable for a continuous production, with extremely low operating costs.

The only use of molecular distillation in the process of the present invention makes it possible the following to be obtained:

1) high quality products, also because they are not submitted to a too large number of chemical processes;
2) products at even very high concentrations, which may reach, in case of EPA DHA mixture, a value of 90%, and, in case of DHA alone, 96%; these concentrations are considerably higher than as claimed in corresponding prior patents;
3) products at different titers for different uses, ranging from dietetic-alimentary uses to the typically pharmaceutical use, by simply suitably varying the parameters of molecular distillation only;
4) the process according to the present invention is particularly suitable for an industrial production, contrarily to many of above cited patents, whose implementation from a laboratory level to the industrial level is much more expensive and problematic;
5) a further advantage is that, in case the ethyl esters have to be obtained, such a preparation can be carried out as the last processing step, by converting the acid products, at a suitable concentration and titer, into the corresponding esters. This is a considerable advantage from the view point of the reduction of the industrial production costs, and furthermore provides a process which is not disclosed in any of prior patents;
6) the process of the present invention is furthermore ideal for an industrial production, because, if the necessary equipment is available, it can be carried out in continuous mode, with a minimum use of labour and at the highest production level.

The high titers (up to 90%) and the considerable degree of purity of the EPA/DHA mixtures obtained by means of the process according to the present invention make it possible to enhance the pharmaceutical effects, which derive from the administration of poly-unsaturated acids of ω3 series, and, in particular, of EPA/DHA.

Having high concentrations of EPA/DHA, on one hand lower-weight, smaller-size pharmaceutical forms can be prepared, which are easier to ingest or administer, and, on the other hand, the number of daily intakes or administrations can be reduced.

The typical characteristics of EPA/DHA products of the present invention make it hence possible a greater therapeutical and formulation advantage to be attained in hyperlipemiae and therewith correlated pathologies, in thromboses, in platelet agglutination, in cardiac infarction, in hypertension, as anticoagulants, in prevention of atherosclerosis, in cerebral infarction, in lesions and occlusions caused by vasomotor spasms, in diabetes and its complications, in acute and chronic inflammations, in self-immune syndromes, in preventing the side effects at gastroenteric level of non-steroid anti-inflamratory agents, in tumor prevention.

The ratio of EPA concentration to DHA concentration changes according to the natural contents of the organism from which both compounds are extracted (e.g., various fish species, fish oils, crustaceans, sea weeds, and so forth).

The therapeutical properties of mixtures prevailingly containing EPA/DHA, or of mixtures containing, besides other poly-unsaturated fatty acids, also EPA/DHA, have been described in the past in several patents, and in particular: in the treatment of thromboses, in hypercholesterolemiae, in myocardial ischemia (WO 84/03899), in the prevention of arteriosclerosis, in cerebral infarction, in hyperlipemiae, in cardiac infarction (EP-Al-O 228 314), in the prophylaxis of atherosclerosis, as antithrombotic, as antihypertensive (JP 62-091188), in thrombotic pathologies, in platelet agglutination, in self-immune syndromes, in acute and chronic inflammations, in atherosclerosis, cardiac infarction, in venous thromboses, in hyperlipemic states, in hypertension, in lesions and occlusions originated by vasomotor spasms, in diabetes (WO 87/02247), in the prevention of the side effects of non-steroid anti-inflammatory agents (EP-Al-0 195 570), in the prophylaxis and management of diabetes complications (JP 60-248610), in hypercholesterolemiae, in hypertriglyceridemiae (DE 34 38 630); as anticoagulants, in hypercholesterolemiae (BE 899 194). Furthermore, both EPA and DHA have an influence on the metabolism of poly-unsaturated fatty acids, promoting the formation of products endowed with a high biological activity, i.e., the ecosanoids, which are active in tumor prevention.

Such activities were evidenced by prevailingly using poly-unsaturated fatty acids of ω3 series, precursors of EPA and DHA (JP 57-187397 and BE 897 806).

The preparations, whose references have been hereinabove cited, are often true mixtures of poly-unsaturated fatty acids prevailingly belonging to ω3 series, and however, the EPA/DHA concentrations used are always considerably lower than those reached by means of the process according to the present invention.

DHA, a highly unsaturated, long-chain fatty acid, belongs to the series denominated as "ω3". Differently from what occurs in lower animal species, wherein both eicosahexaenoic acid (EPA) and DHA are present, in man only traces of EPA, and high concentrations of DHA are found.

DHA is present in exclusively esterified form in membrane glycerophospholipids, and, in particular, in some districts, such as the CNS, in synaptic membranes and in retinal cells.

To the poly-unsaturated fatty acids belonging to ω3 series, and to EPA, metabolic precursors of DHA, an extremely high number of biological and therapeutical activities have been attributed.

In the metabolic pathway starting from α-linolenic acid and leading to DHA, the administration of EPA does not lead, except for small amounts, to the conversion into DHA, whilst a portion of administered DHA is converted back into EPA.

In fact, the ingestion of DHA, in ester form, and/or as the free acid, significantly increases both DHA and EPA levels in plasmatic phospholipids (Hiroi at al., 1978).

Thus, DHA, besides performing its own task, would also ensure, by being converted back into EPA, the biological actions typical of EPA.

In prior patents, several therapeutical activities have been claimed for mixtures of poly-unsaturated fatty acids of ω3 series, to which DHA belongs, and, in particular, therapeutical activities have been claimed in hyperlipemiae and therewith correlated pathologies, in thromboses, in platelet agglutination, in cardiac infarction, in hypertension, as anticoagulants, in atherosclerosis prevention, in cerebral infarction, in lesions and occlusions caused by vasomotor spasms, in diabetes and its complications, in acute and chronic inflammations, in self-immune syndromes, in preventing the side effects at the gastroenteric level of non-steroid anti-inflammatory agents, and in tumor prevention (WO 87/03899, EP-Al-0 228 314, JP 62-091188, WO 87/02247, EP-Al-0 195 570, JP 60-248610, DE 34 38 630, BE 899 184, JP 57-187397, BE 897 806).

DHA, as a single substance, was evaluated in therapy as a platelet agglutination preventive agent, and an use thereof in the prophylaxis of thrombotic processes was proposed (GB 2,098,065, GB-2,090,529).

In reality, the DHA used in the prior studies does not seem to have been as highly concentrated as DHA obtained by means of the process according to the present invention.

Furthermore, the process of extraction of present invention, by means of molecular distillation, without either chemical or physical treatments, characterizes the obtained DHA with a high purity degree, as compared to the previously obtained products.

By means of different experimental models, the activity of highly concentrated (96%) and purified DHA in hyperlipemiae was pointed out. In fact, the administration of DHA reduced, to a meaningful extent, the experimentally induced high levels of cholesterol and triglycerides.

On considering the obtained results, on the basis of the functions which DHA performs inside the organism, and as a consequence of the phenomena observed in various districts when DHA is administered, its characteristics and therapeutical peculiarities can be summarized as follows: in the treatment and prophylaxis of dislipemic diseases and therewith connected pathologies, such as hyperlipoproteinemiae, hypercholesterolemiae, hypertriglyceridemiae, in the alterations of fat metabolism, in damages to vessels caused by cholesterol, in atherosclerosis, in xanthomas, in diabetic retinopathy, in the prevention of thrombus formation, in prevention of aortal and coronary arteriosclerosis, as a coadjuvant in those diseases which may originate manifestations of hyperlipoproteinemiae (diabetes mellitus, hypothyroidism, uraemia, and so forth), in cardiac infarction, in platelet agglutination, in hypertension, in anticoagulant therapy, in cerebral infarction, in acute and chronic inflammations, in diabetes, in self-immune syndromes, in the prevention of the side effects caused by non-steroid anti-inflammatory agents, in tumor prevention, in retinopathies with visual deficit, in ceroidoses, in the processes relevant to learning and ageing.

The process according to the present invention is disclosed now in detail, and one will thus see that by means of said process, those purposes and advantages which have been hereinabove outlined can be fully achieved.

An alkaline hydrolysis (NaOH) of the raw oil is performed up to the complete breakdom of the triglycerides.

The solid soap formed is collected and is immediately acidified with mineral acid in an aqueous solution.

The formed acids are extracted with petroleum ether, un to exhaustion. The extracts are combined with one another, are thoroughly washed with water, and are concentrated up to total solvent removal.

The resulting product is processed by exclusively using molecular distillation, in such a way as to obtain the whole range of products according to the present invention.

A) COMPLEX CONSTITUTED BY EPA AND DHA

A - 1 Total concentration comprised within the range of from 35 to 40%

A - 2 Total concentration comprised within the range of from 40 to 50%

A - 3 Total concentration comprised within the range of from 50 to 60%

A - 4 Total concentration comprised within the range of from 60 to 70%

A - 5 Total concentration comprised within the range of from 70 to 80%

A - 6 Total concentration comprised within the range of from 80 to 90%

B) PRODUCT CONSTITUTED BY DHA

B - 1 Total concentration 90%

B - 2 Total concentration 96%

- Process for obtaining A-1 product

The mixture of fatty acids obtained from the first step of the process (saponification) is submitted to molecular distillation, operating under a pressure of 0,133 Pa, with a temperature of the evaporator of 110-120°C, in order to remove the process impurities and the natural impurities, which constitute the residue.

The distillate maintains the concentration of EPA and DHA which was originally present in the starting oil (15-20% of EPA and 15-20% of DHA, in case of a fish oil), and is free from abobe-said impurities; it constitutes, per se, the A-1 end product, and is the starting material for subsequent A-2, A-3, A-4 products.

- Process for obtaining A-2 product

A-1 product is submitted to molecular distillation, under a pressure of 0,133 Pa and with an evaporator temperature of 50°C. The residue is constituted by A-2 product, and the increase in EPA and DHA titer takes place to the detriment of the lower molecular-weight acids ($C_{16}$ and $C_{18}$), which constitute the distillate.

- Process for obtaining A-3 product

A-1 product is submitted to molecular distillation under the above-said conditions, except for evaporator temperature, which is increased to 60°C. The residue is constituted by A-3 product, and the increase in EPA and DHA titer takes place to the detriment of the lower molecular-weiqht acids ($C_{16}$ and $C_{18}$), which constitute the distillate.

- Process for obtaining A-4 product

A-1 product is submitted to molecular distillation under the above-said conditions, except for evaporator temperature, which is increased to 70°C. The residue is constituted by A-4 product, and the increase in EPA and DHA titer takes place to the detriment of the lower molecular-weight acids ($C_{16}$ and $C_{18}$), which constitute the distillate.

- Process for obtaining A-5 product

A-4 product is submitted to molecular distillation under the above-said conditions, except for evaporator temperature, which is increased to 75°C. The residue is constituted by A-5 product, and the increase in EPA and DHA titer takes place to the detriment of the lower molecular-weight acids ($C_{16}$-$C_{18}$ and lower-unsaturated-$C_{20}$), which constitute the distillate.

- Process for obtaining A-6 product

A-5 product is submitted to molecular distillation under the above-said conditions, except for evaporator temperature, which is of 80°C. The residue is constituted by A-6 product, and the increase in EPA and DHA titer takes place to the detriment of the lower molecular-weight acids ($C_{16}$-$C_{18}$ and lower-unsaturated-$C_{20}$), which constitute the distillate.

- Process for obtaining B-1 product

A-6 product is submitted to a double molecular distillation under the above-said conditions, except for evaporator temperature, which is of 85°C. The residue is constituted by B-1 product (DHA 90%), and the distillate is mainly constituted by EPA and minor amounts of other acids.

- Process for obtaining B-2 product

B-1 product is submitted to molecular distillation under the same condition as used for B-1, with the evaporator temperature being of 85°C. The residue is constituted by 96% of DHA, and the distillate is mainly constituted by EPA and minor amounts of other acids.

DHA acts, through several mechanisms, on some metabolic processes and body districts, favouring a precise pharmacological placing thereof in some pathologies. It is important that preparations of DHA are used, which contain at least 5-10% of alpha-tocopherol, in order to prevent phenomena of peroxidation to the detriment of DHA, which is easily subject to oxidative processes, owing to the high unsaturation level thereof.

1) Action of DHA on metabolism of poly-unsaturated fatty acids.

In nature, several families of fatty acids exist, to each of which compounds belong, which are correlated with one another from a metabolic standpoint.

The three main families of poly-unsaturated acids are constituted by those compounds which belong to n-9, n-6 and n-3 metabolic series. By means of each of said short names, fatty acids are meant, which are endowed with the characteristic of respectively having the nearest double bond to methyl end (and simultaneously most far away from carboxy end) at a distance of 9, 6 and 3 C atoms.

Inasmuch as the fatty acid molecule portion comprised between the methyl end and the nearest double bond to it is not modified during the metabolic transformations (desaturation and chain extension) which the molecule can undergo, it derives that all compounds formed by metabolic interconversion maintain unchanged this structural characteristic. The respective parent compounds of the mentioned metabolic series of fatty acids are oleic acid, linoleic acid and alpha-linoleic acid, to which DHA belongs.

Linoleic acid and alpha-linoleic acid cannot be synthetized in upper organisms.

Such compounds, which play important biological roles in upper organisms, are hence essential compounds from a nutritional viewpoint, and must be intaken by means of food.

The reactions of metabolic conversion of the above indicated fatty acids into longer-chain, higher-unsaturated compounds take place by means of the activity of desaturation enzymes (desaturases) and chain-extending enzymes (elongases), prevailingly located at liver level (reticuloendoplasmatic system).

The following considerations are important, as relates to the metabolism of the various series of poly-unsaturated fatty acids.

a) Regulation steps for such reactions axist, and, in particular, the desaturation reactions are limiting steps.

b) The speeds of conversion of the precursors of the three series of fatty acids are very different from one another; such a speed is much higher for alpha-linoleic acid 18:3 (n-3 series), much lower for linoleic acid (18:2, n-6), and minimum for oleic acid.

c) A competitive antagonism exists in the metabolism of the three series of poly-unsaturated fatty acids, with the consequent inhibition, by the larger-affinity acids, of the metabolic conversion of the lower-affinity acids. On the contrary, the lack in the diet of acids with a high affinity for the enzymatic systems (e.g., the lack in essential linoleic and alpha-linoleic acids) unb locks the conversion of lower-affinity acids (oleic acid).

Therefore, in case of deficiencies of essential fatty acids, a conversion of oleic acid up to eicosatrienoic acid, C 20:3 n-9 takes place.

d) As a consequence of the metabolic interactions between the various unsaturated fatty acids (n-3, n-6, n-9) supplied with the diet, it happens that the mutual ratio thereof conditions the metabolic conversion of $C_{18}$-compounds belonging to the various series into more-unsaturated, longer-chain compounds, and, consequently, their incorporation with plasrmatic and tissular lipids.

The administration of poly-unsaturated acids of n-3 series, such as DHA, is hence very important as to their metabolic use, and their incorporation with the tissues.

In fact, it is evident that the administration, e.g., of DHA, will cause an incorporation of such compound with the cellular lipids, which will depend on several factors, such as the relative levels of n-6 acids in the diet, besides the relative affinity of DHA, as compared to that of n-6 acids, for the various phospholipidic pools in different cellular types.

2) Action of DHA on vascular district, on atherosclerosis, platelet functions and thrombus formation.

As a consequence of what reported above on the influence of DHA on the metabolism of unsaturated fatty acids, the role is important which it may play on $C_{20}$ poly-unsaturated fatty acids, precursors of products endowed with a high biological activity, viz., eicosanoids.

Eicosanoids are substances prevailingly deriving by enzymatic oxygenation from arachidonic acid (AA), through the following two main routes: cyclooxygenase, leading to the formation of prostaglandins, prostacycline and thromboxane, and lipoxygenase, leading to the formation of hydroxyacids and leukotrienes.

Some specific eicosanoids may take an action in the mechanisms which regulate important functions, and which constitute the basis for some processes, such as in the formation of thrombi, and in vascular district (activation of production of $I_3$ prostacycline, or $PGI_3$, instead of $PGI_2$).

Through the formation of the specific eicosanoids and the consequent production of inactive $TxA_3$, DHA also acts on platelet agglutination (Rao G.H.R. et al., Biochem.

Biophys. Res. Comm., 117, 549, 1983). However, the effects on some systems of the administration of purified DHA are rather different from the effects exhibited by EPA.

In fact, although after DHA administration a platelet agglutination preventive activity takes place, no particular interferences with the metabolism of AA were observed, differently from what occurs when EPA is administered (Hirai et al., in "advances in Prostaglandins, Thromboxane and Leukotriene Research", Vol. 17, Eds. Samuelsson B., Paoletti R. and Ramwell P.W., Raveb Press, N.Y., page 838, 1978). Such data suggests that DHA incorporated with platelet lipids is neither easily released, nor, consequently, converted into other compounds.

Furthermore, DHA, according to the studies by Talesnik and Carleton (Talesnik J, and Carleton Hsia J., Eur. J. Pharmacol., 80, 255, 1982), inhibits, at a coronaric level, the vasoconstriction induced by AA.

It derives therefrom that DHA, through direct conversion, or reconversion into EPA with intervention on the metabolism of $C_{20}$-fatty acids, can lead to the formation of specific eicosanoids, with the possibility of acting, at a preventive level, or at a therapeutical level, as an antithrombotic, in extending the bleeding times, as a coronary vasodilator, and as a platelet agglutination inhibitor.

3) Action of DHA on immunity system and in inflammation

The fatty acids belonging to n-3 series, to which DHA belongs, play an important role in varying the immunity and inflammatory responses, through the modifications in the cellular poly-unsaturated fatty acids, with consequent changes in the synthesis of prostaglandins and leukotrienes, in the cells engaged in the immunity and inflammatory responses (leukocytes, monocytes, T and B lymphocytes).

DHA performs an action in the inflammatory process on the synthesis of prostaglandins through the competitive inhibition of the conversion of arachidonate into $PGE_2$, as it occurs for some non-steroid anti-inflammatory agents, such as indomethacin (Corey E.J. et al., Proc. Natl. Acad. Sci., 80, 3581, 1983).

Furthermore, various other processes of cellular activation can be modified by changes in the poly-unsaturated fatty acids in structural membrane lipids, as a consequence of the administration of fatty acids of n-3 series. Among these, an important role has to be assigned to the mobilization of intracellular Ca after stimulation, and to the generation of inositol phosphates from membrane phosphoinositide pools.

4) Action of DHA on visual functionality, ceroidosis, in learning and ageing processes

High concentrations of DHA are contained in retina and in synaptic terminations.

The depletion of DHA in such structures, obtained by means of dietetic manipulations in laboratory animals, causes visual malfunctions. (Tinoco J. et al., Biochim. Biophys. Acta, 486, 575, 1977).

Therefore, the availability of highly purified DHA in the treatment of those pathologies correlated with a decrease in DHA concentrations in retinal glycerophospholipids seems to be highly interesting from a therapeutical viewpoint.

DHA depletion causes alterations in behaviour, with learning deficit. In fact, as Lamptey and Walker were able to observe (Lamptey M.S. and Walker B.L., J. Nutr., 106, 86, 1976) the learning capability shown by rats submitted to diets with different added amounts of n-3 acids was directly proportional to cerebral levels of 22:6 (DHA). Particularly interesting is also the correlation between ceroidosis and DHA concentration.

Ceroidosis is a pathology wherein a lipofucsinic pigment of brown colour (ceroid) can deposit inside cells of smooth-muscles of digestive tube, in liver, in muscles and in CNS.

Pullarkat et coworkers (Pullarkat R.K. et al., Neuropädiatrie, 9, 127, 1978) evidenced that in juvenile neuronal ceroidosis a correlation exists between the decrease in leukocytic DHA, found in all examined patients, and the seriousness of the disease.

Furthermore, the same Authors observed, in a prior study, a decrease in DHA content in phosphatidylserine present in cerebral gray in adult and juvenile forms of neuronal ceroidosis.

The supply of exogenous, highly-purified DHA may be regarded as a valuable therapeutical means in the evolution of such a pathology.

As reported in the foreword, DHA is found in upper animals in ester form in membrane glycerophospholipids.

Among main tissular glycerophospholipids (phosphatidylcholine, PC or lecithin; phosphatidyl-ethanolamine, PE; phosphatidylserine, PS; and phosphatidyl-inositol, PI), and, in particular, PE and PS at a cerebral level, contain relatively high levels of DHA. The relative concentrations of DHA, relatively to total fatty acids, at the level of membranes of preparations of synaptosomes may reach values as high as 20%, with a ratio of >1 relatively to AA, the main poly-unsaturated acid present in other tissues. Said two phospholipids, PE and PS, are localized on the inner cellular membrane surface, and therefore the high DHA levels in such phospholipids suggest that such a fatty acid may be involved in some functions inside the cell. It is also interesting to observe that PC and PI, at the cerebral level, either contain very low DHA levels, the first one, or do not contain any DHA at all, the second one (Galli C. et al., in "Advances in Prostaglandins and Thromboxane Research", vol. 4, Eds. Coceani F. and Olley P.M., Raven Press, N.Y., page 181, 1978).

In the same work, the particular abundance of 22:6 (DHA) in phospholipids of synaptosomial membranes is observed. The administration of a diet containing an oil rich in n-3 acids, and in particular of DHA, such as a fish oil, causes a sharp increase of this poly-unsaturated acid in membrane lipids.

Also in man (White H.R. et al., J. Neurochem. 18, 1337,1971) decrease at cerebral level during the course of ageing.

In lipids isolated from cerebral synaptosomes of aged rats, a significant decrease in DHA is observed as compared to the values which are observed in adult rats.

Studies on rat also demonstrated that the capability of incorporating DHA administered by means of the diet, with cerebral lipids, considerably decreases during ageing (Eddy D.E., Harman D., J. Am. Ger. Soc. 25, 220, 1977).

These Authors postulated that such a decrease is due to an increase in lipid peroxidation at the cerebral level during the course of ageing.

Therefore, the possibility of being able to use concentrated DHA in all those complex mechanisms which lead to the learning and ageing process seems to be of particular moment.

Furthermore, of considerable interest appears to be the fact that several proprietary medicines used in medical practice contain cerebral phospholipids, the natural seat of DHA, with the following operating mechanisms and indications.

Phosphatidylserine: influence on the parameters of cerebral metabolism, altered during ageing. Therapeutical applications in chronic cerebral psycho-organic syndromes and valuable use in therewith correlated symptoms (lack in memory - confusion - poor attention and concentration, emotional lability, irritability, depressed mood, anxiety).

Diencephalic phospholipids: The liposomes of hypotalamus phospholipids are capable of activating the hypotalamus metabolism, increasing dopamine turnover, the activity of tyrosine-hydroxylase and of adenylcyclase, with a consequent increase in cyclic AMP. This effect reflects itself in particular on the functionality of hypotalamushyponhysis axis. Application as a coadjuvant in cerebral metabolic alterations consequent to neuroendocrine disorders such as depressive syndromes, anxious-depressive statuses occurring during developmental age, in climacteric syndrome and in hypoprolactinemiae.

Cerebral phospholipids: are capable of activating the neuronal metabolism, normalizing the enzymatic activities of membranes, increasing the neurotransmitters turnover. Therapeutical application in neurologic syndromes such as arteriosclerotic cerebrovascular pathologies, involution syndromes, parkinsonian syndromes, cranio-encephalic traumatic lesions and psychosomatic hypoevolutism, as well as in all pathologies connected with altered statuses of CNS metabolism.

5) Action of DHA on cardiac functionality

The interest in the favourable effects of n-3 acids on various biologic parameters above all of cardiovascular district arouse nearly ten years ago.

The key observations which played a determining role in promoting this interest were some epidemiological studies carried out on populations (in particular the Eskimos) consuming very large amounts of fish, very rich in DHA, which evidenced an extremely low incidence of cardiovascular pathologies, notwithstanding the high contents of fats in their diet.

On rat, it was observed that the metabolism and the function at the cardiac level, in animals submitted to diets with different compositions as for fatty acids, are correlated with the contents of DHA in cardiac phospholipids (Gudbjarnason S. et al., Acta Biol. Med. Germ., vol. 37, 777, 1978).

In fact, the exposure to catecholamine-stress involved the replacement of linoleic acid by DHA in cardiac lipids, whilst cardiac frequency in groups of animals submitted to different diets was proportional to the DHA contents of the same lipidic fractions.

The therapeutical properties and prevention capabilities of DNA, in certain cardiac diseases, are probably also bound, besides a direct action, to the different activities thereof in such different compartments as anti-atherosclerotic activity, antithrombotic activity, hypolipemic activity and platelet agglutination preventive activity.

6) Action of DHA on hyperlipemiae

Hyperlipoproteinemic pathology is a condition wherein the concentration of cholesterol or of the lipoprotein-bearing triglycerides in plasma exceeds the normal physiologic limits.

At present, the percentage of population affected by high values of plasmatic concentration of cholesterol and/or triglycerides is estimated to be around 95% of total.

These limits vary according to age and sex.

Hyperlipoproteinemiae can be subdivided into primary and secondary hyperlipoproteinemiae.

Primary hyperlipoproteinemiae may be subdivided into two main groups: the monogenic primary hyperlipoproteinemiae (of genetic origin) and the polygenic primary hyperlipoproteinemiae (probably in already predisposed individuals, with the addition of incorrect diets and obesity).

The secondary hyperlipoproteinemiae evidence themselves as complications of metabolic disturbances in some pathologies, as diabetes mellitus, hypothyroidism, uremia, overdrinking, or as secondary effects in the use of oral contraceptives in subjects genetically prone to hypertriglyceridemia.

It is ascertained by now on scientific grounds that high concentrations of lipoproteins accelerate the development of arteriosclerosis, which may subsequently cause such irreparable damages as thrombosis and cardiac infarction.

According to computations, in the U.S.A. approximately half deceases are likely to be due to such events.

With the evolution of industrialized civilization and with the relevant increase in social welfare, people, especially over past decades, started to overindulge in food, both as regards the amounts, and, from the dietetic-nutritional viewpoint, the quality.

In fact, the intake hase progressively increased of food rich in cholesterol and saturated fatty acids.

This is one of the main causes of the increase in dislipemic pathologies.

The first therapeutical aid for all hyperlipoproteinemiae is the use of a diet which maintains a normal body weight and reduces the concentrations of lipids in blood.

The elimination from the diet of saturated animal oils, to be replaced by poly-unsaturated vegetable oils, is apriority condition for lipidic illnesses to show a positive evolution.

Due to this reason, we took into consideration the possibility that DHA, a poly-unsaturated long-chain fatty acid, may perform an action in dislipemic pathology.

Therefore, some preliminary pharmacologic tests were developed, in order to evidence the possible activity of DHA, by oral way, in test animals.

In all tests, Sprague-Dawley albino rats were used.

Esterified highly-concentrated DHA, having a titer of 96%, was used.

A group of animals per each test were treated with clofibrate or nicotinic acid, well-known molecules endowed with hypolipemic activity, in order to verify the experimental validity.

All animals, at sacrifice time, were under fasting conditions.

A) Activity of DHA in hyperlipemia induced by Nath diet.

Nath diet is a diet above all rich in cholesterol and cholic acid and, when intaken for a 4-5 weeks time, causes hyperlipemia in the animal.

Male rats of an initial weight of approximately 80-100 grams were used.

The animals were subdivied into 6 experimental groups:

1) controls (C);
2) controls + Nath diet (CD);
3) clofibrate, 300 mg/kg daily + Nath diet (CL);
4) DHA, 50 mg/kg daily + Nath diet;
5) DHA, 150 mg/kg daily + Nath diet;
6) DHA, 500 mg/kg daily + Nath diet.

The test lasted 5 weeks, during which the general conditions of the animals (growth, food consumption, etc.) were monitored.

At test end, the animals were sacrificed under fasting conditions, in order to evaluate the parameters (total cholesterol, triglycerides)

The results obtained confirm that the administrations of highly concentrated DHA inhibit the hyperlipemic effects experimentally induced by the nourishing with the Nath diet (Figure 1).

B) Activity of DHA on hyperlipemia induced by Triton WR 1339.

Triton WR 1339 or Tyloxapol is a substance capable of increasing the hematic levels of triglycerides and cholesterol in the laboratory animal.

Male rats of approximately 200 g of initial weight were used.

The animals were subdivided into 6 experimental groups:

1) controls (C);
2) controls + Triton (CT);
3) nicotininc acid, 500 mg/kg daily + Triton (AN);
4) DHA, 50 mg/g daily + Triton;
5) DHA, 150 mg/kg daily + Triton;
6) DHA, 500 mg/kg daily + Triton.

The animals, before receiving Triton WR 1339, were pre-treated for a 2-days period with nicotininc acid, DHA or with the carrier only.

Eighteen hours after the treatment with Triton WR 1339, the animals, under fasting conditions, were sacrificed for the evaluation of the hematic levels of total cholesterol and triglycerides.

In this test too, the administration of DHA evidenced capability of this compound of decreasing the levels of the plasmatic lipids altered by the treatment with Triton WR 1339 (Figure 2).

C) Activity of DHA in hypertriglyceridemia induced by ethanol

The subacute administration of ethanol causes, in rat, a condition of hypertriglyceridemia.

For this test, male rats of an initial weight of approximately 200 grams were used.

The animal were subdivided into six test groups:

1) controls (C);
2) controls + ethanol (CE);
3) clofibrate, 200 mg/kg daily + ethanol (CL);
4) DHA, 50 mg/kg daily + ethanol;
5) DHA, 150 mg/kg daily + ethanol;
6) DHA, 500 mg/kg daily + ethanol.

Oral administrations of ethanol ad libitum in solution at 10%, alternating with administrations of 0.5 g/rat, induce hypertriglyceridemia in rat.

Since the day prior to the first intake of ethanol, the animals were treated with the substances under test. On the 4th day, 2 hours after the last treatment with ethanol, the animals were sacrificed in order to determine the triglycerides.

In this test, very reassuring results were obtained.

In fact, the oral administration of high-concentration DHA significantly inhibited hypertrigliceridemia induced by ethanol (Figure 3).

Summing up, on considering the results obtained in the experimental tests, the use of high-titer DHA (96%) in the therapy of hyperlipemic pathologies and in the therewith correlated pathologies can be regarded as at all justified.

## Claims

1. Process for preparing high-concentrated mixtures of eicosapentaenoic acid (EPA) and docosahexaenoic acid (DHA) and their esters, from oils of animal and/or vegetable origin, characterized in that the raw oil is submitted to an alkaline hydrolysis, the solid soap so formed is acidified with a mineral acid in aqueous solution, the resulting mixture is extracted with petroleum ether up to exhaustion and then, after washing and concentration with total solvent removal, the combined extracts are submitted to one or more molecular distillation step(s), with the pressure and temperature parameters being suitably changed, in order to obtain the whole range of the desired end products, the first molecular distillation being carried out under a pressure of 0.133 Pa, and at a temperature of the evaporator of approximately 110-120°C, the so obtained first distillate having a concentration of 15-20 % of EPA and 15-20 % of DHA.

## Patentansprüche

1. Verfahren zur Herstellung hochkonzentrierter Mischungen von Eicosapentaensäure (EPA) und Docosahexaensäure (DHA) und ihrer Ester aus Ölen tierischen und/oder pflanzlichen Ursprungs, dadurch gekennzeichnet, daß das Rohöl einer alkalischen Hydrolyse unterworfen wird, die so gebildete feste Seife mit einer Mineralsäure in wässriger Lösung angesäuert wird, die resultierende Mischung mit Petroläther bis zur Erschöpfung extrahiert wird und dann nach Waschen und Konzentrierung unter vollständiger Entfernung des Lösungsmittels die vereinten Extrakte einem oder mehreren Molekular-Destillationsschritt(en) unterworfen werden, indem die Druck- und Temperaturparameter in geeigneter Weise verändert werden, um den gesamten Bereich der gewünschten Endprodukte zu erhalten, die erste Molekulardestillation bei einem Druck von 0,133 Pa und bei einer Verdampfertemperatur von ungefähr 110-120° C durchgeführt wird, das so erhaltene erste Destillat eine Konzentration von 15-20 % von EPA und 15-20 %. von DHA hat.

## Revendications

1. Procédé de préparation de mélanges très concentrés d'acide eicosapentaénoïque (EPA) et d'acide docosahexaénoïque (DHA) et de leurs esters, à partir d'huiles d'origine animale et/ou végétale, caractérisé par le fait que l'huile brute est soumise à une hydrolyse alcaline, le savon solide ainsi formé est acidifié par un acide minéral en solution aqueuse, le mélange résultant est extrait par de l'éther de pétrole jusqu'à épuisement, puis, après lavage et concentration avec élimination totale du solvant, les extraits combinés sont soumis à une ou plusieurs étape(s) de distillation moléculaire, les paramètres pression et température étant modifiés de façon appropriée, de façon à obtenir la gamme totale des produits finals désirés, la première distillation moléculaire étant effectuée sous une pression de 0,133 Pa, et à une température de l'évaporateur d'approximativement 110-120°C, le premier distillat ainsi obtenu ayant une concentration de 15-20% d'EPA et de 15-20% de DHA.

Fig. 1 - Effect of the administration of highly purified (96 %) DHA on rats affected by hypercholesterolemia and hypertriglyceridemia induced by Nath diet (preliminary data)

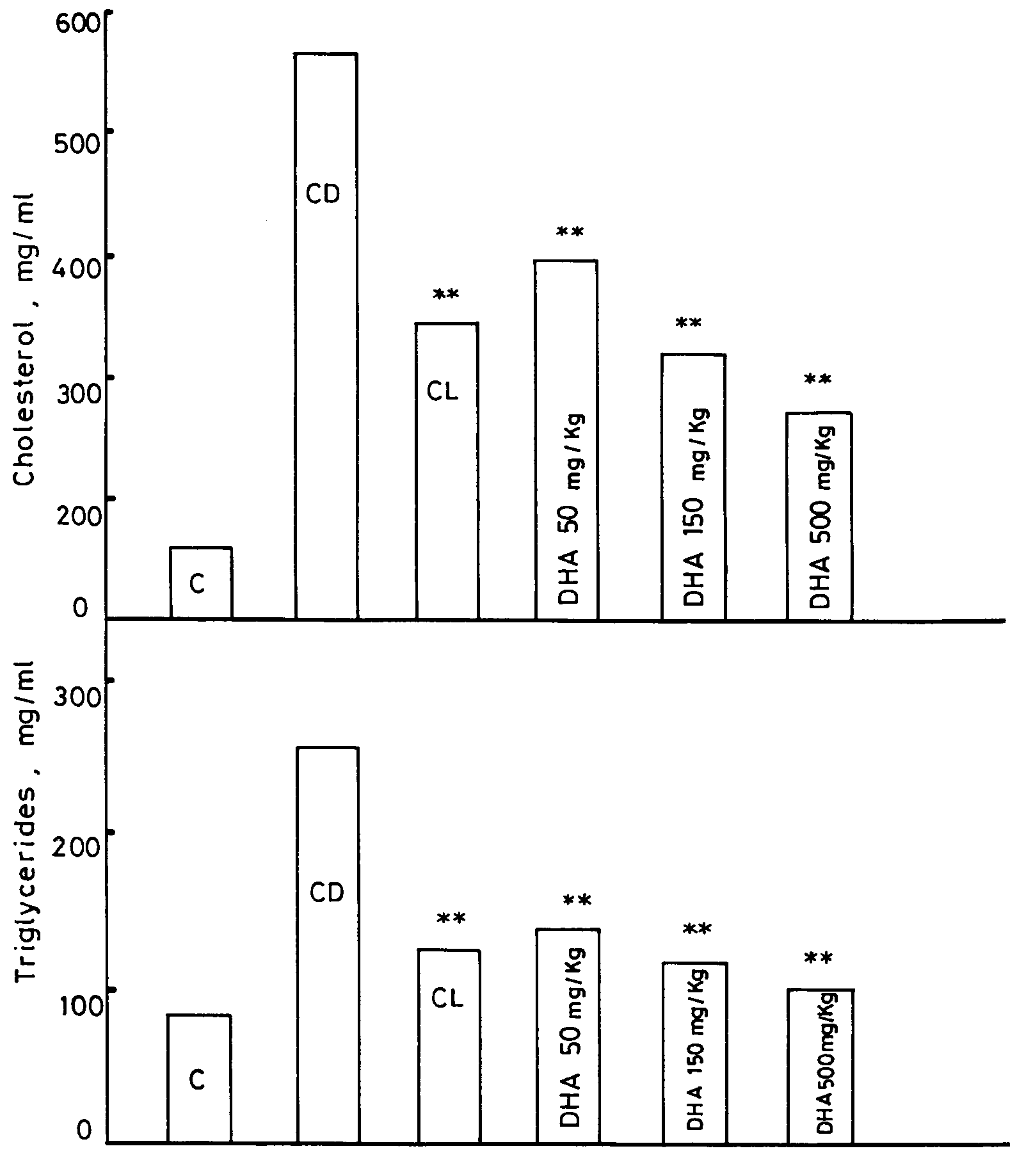


** P < 0.01 vs CD

Fig. 2 - Effect of the administration of highly purified (96 %) DHA on rats affected by hypercholesterolemia and hypertriglyceridemia induced by WR 1339 (preliminary data)

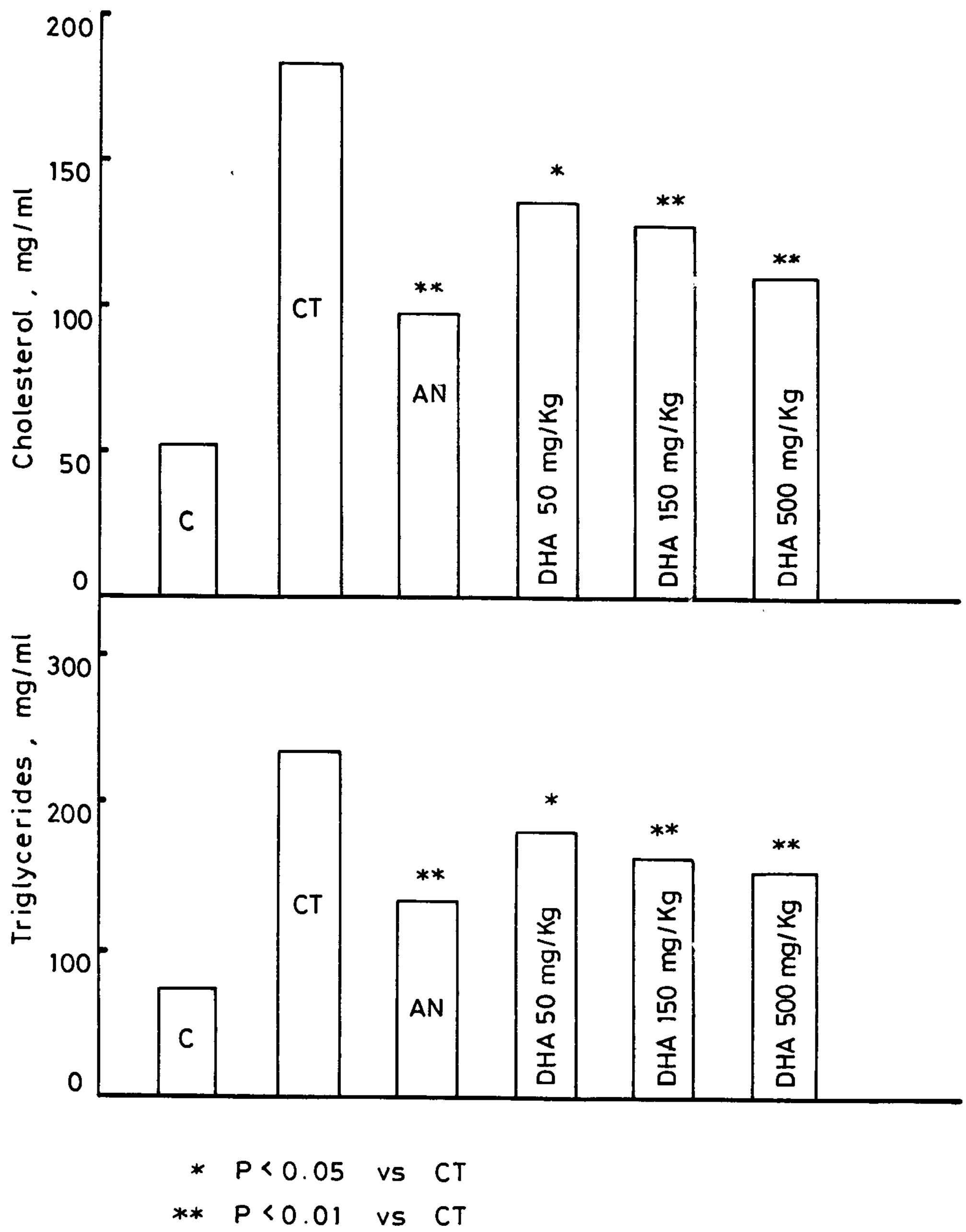

Fig. 3 - Effect of the administration of highly purified (96 %) DHA on rats affected by hypertriglyceridemia induced by ethanol (preliminary data)

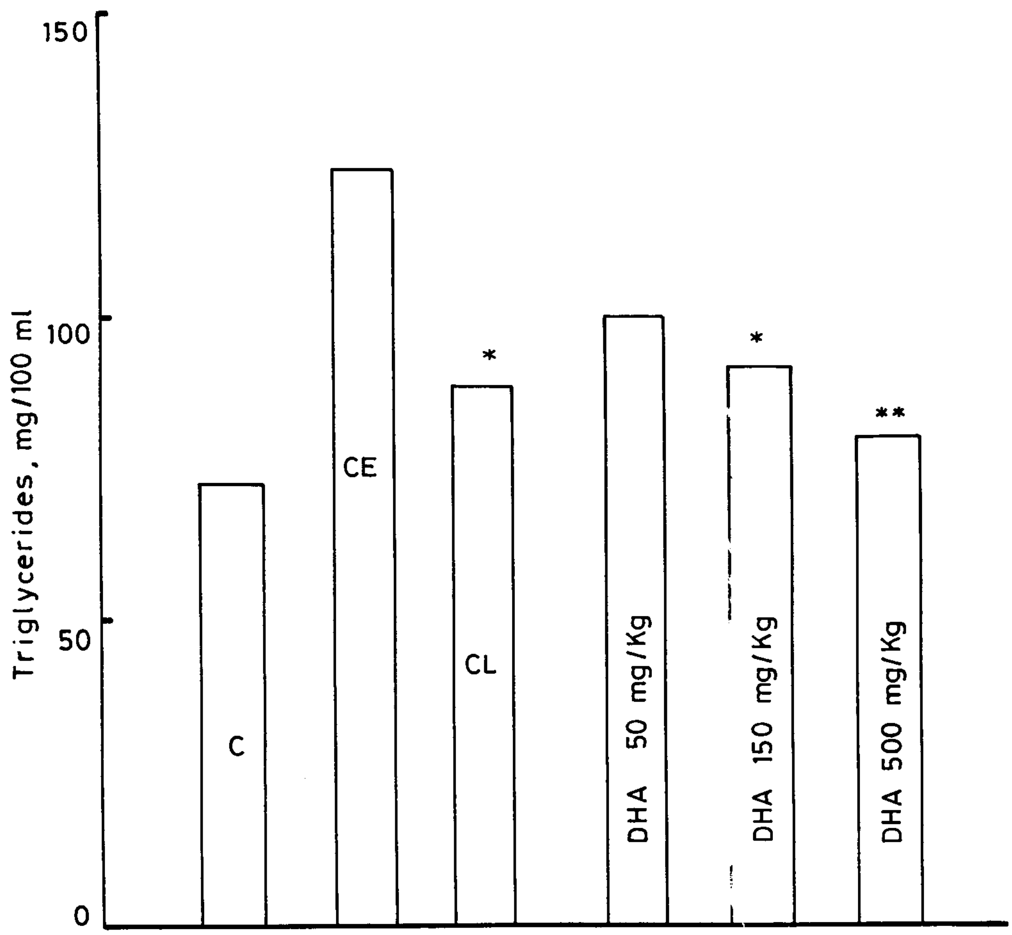


* P < 0.05 vs CE
** P < 0.01 vs CE